# EUROPEAN PATENT APPLICATION

(11) **EP 2 592 158 A1**
(43) Date of publication of application: **15.05.2013**
(21) Application number: 11189012.5
(22) Date of filing: 14.11.2011
(51) Int. Cl.: C12R 1/25, C12N 1/20, A61K 35/66, A23L 2/42, A23L 3/00, A23C 9/123

(54) **Lactobacillus plantarum species possessing broad spectrum anti-fungal activity and exhibiting high heat tolerance and osmotolerance**

(71) Applicant: University College Cork, National University of Ireland, Cork, Cork (IE)
(72) Inventor: Van Sinderen, Douwe, Co. Cork Carrigrohane (IE); Crowley, Sarah, Waterford Dunmore (IE)
(74) Representative: Purdylucey Intellectual Property

(57) **Abstract**

Isolated strains of *Lactobacillus plantarumare* described that have anti-fungal activity against fungal spore suspensions, and specifically anti-fungal activity in fruit juices and fermented dairy products. The two species have also been found to have a high degree of heat tolerance and osmotolerance, and to have a high freeze-drying survival rate.

## Description

### Introduction

Fungal contamination of foods prevails as a principal problem for food manufacturers. Food preservation methods such as the use of chemicals or irradiation may impart undesirable properties to foods as well as raise concerns among consumers. Furthermore the production of mycotoxins and the development of chemical resistant fungi are problematic for the food industry. Consequently, the need for alternative "consumer friendly" preservation methods has become a focal point for the food sector.

The application of LAB that produce antifungal activity is a promising alternative to the use of chemical preservatives due to their GRAS (Generally Regarded As Safe) status and widespread exploitation in various food fermentations. The number of reports concerning antifungal LAB, the inhibitory compounds they produce and their application as food biopreservatives has expanded in recent years. An array of antifungal compounds from LAB have been described including proteinaceous compounds, cyclic dipeptides, hydroxyl fatty acids, phenyllactic acids and bacteriocin-like substances. More recently, novel compounds such as C₁₂H₂₂N₂O₂ ,3,6-bis(2-methylpropyl)-2,5-piperazinedion and dodecalacetone and have been associated with fungistatic activities. The application of LAB as antifungal agents in foods has also been well documented in recent years. Antifungal-producing isolates of LAB have been successfully applied in the preservation of a variety of foods such as cheese, cucumbers, apples, corn and soybeans. The preservation of breads using antifungal LAB has also been documented. Ryan *et al.* (2011) recently reported that sourdough bread fermented with the antifungal strain *Lactobacillus amylovorus* DSM 19280 possessed an extended shelf life of two weeks compared to control breads fermented with a non-antifungal strain or those treated with calcium propionate. In addition, sourdough fermented with *Lb. plantarum* lA7 (S1A7) did not exhibit fungal contamination until seven weeks storage. The aerobic quality of silage can also be improved by the addition of *Lb. buchneri* which was shown to reduce yeast counts.

Food spoilage attributable to yeasts represents a significant problem for both the beverage and dairy industries. Their ability to survive at low temperatures and pH levels make them ideal candidates for spoilage of foods such as yoghurts, cheeses and juices. Spoilage yeasts have been associated with a variety of foods including meats and fermented vegetables. Growth of spoilage yeasts in foodstuffs results in deterioration of the optical, physical and organoleptic properties of foods, aside from serious human health implications.

It is an object of the invention to overcome at least one of the above-referenced problems.

### Statements of Invention

The invention is based on the surprising finding that two specific species of *Lactobacillus plantarum, Lb. plantarum 16* and *Lb. plantarum 62*, have anti-fungal activity against fungal spore suspensions, and specifically anti-fungal activity in fruit juices and fermented dairy products. The two species have also been found to have a high degree of heat tolerance and osmotolerance, and to have a high freeze-drying survival rate.

A deposit of *Lb. plantarum 16* was made at the National Collection of Industrial and Marine Bacteria Limited (NCIMB) on 19 October 2011 and accorded the accession number NCIMB41875.

A deposit of *Lb. plantarum 62* was made at the National Collection of Industrial and Marine Bacteria Limited (NCIMB) on 19 October 2011 and accorded the accession number NCIMB41876.

Accordingly, the invention provides an isolated bacterium selected from the group consisting of the following bacteria deposited with the National Collection of Industrial and Marine Bacteria Limited (NCIMB) on 19 October 2011:
- *Lb. plantarum (16)* NCIMB41875, or derivatives thereof; and
- *Lb. plantarum (62)* NCIMB41876, or derivatives thereof.

The invention also relates to a fermentation broth, a cell culture supernatant, or an extract of the bacteria, broth or supernatant, derived from an isolated bacterium of the invention.

The invention also relates to at least one bacterial culture selected from the group consisting of:
- *Lb. plantarum (16)* NCIMB41875 or derivatives thereof;and
- *Lb. plantarum (62)* NCIMB41876, or derivatives thereof,
for use as an anti-fungal agent, typically for use as an antifungal agent in a food product prone to spoilage, especially a food-based food product or a dairy product.

The invention also relates to at least one bacterial culture selected from the group consisting of:
- *Lb. plantarum (16)* NCIMB41875 or derivatives thereof; and
- *Lb. plantarum (62)* NCIMB41876, or derivatives thereof,
for use as a biopreservative, typically for use as a biopreservative in a food product prone to spoilage, especially a fruit-based food product or a dairy product.

The invention also relates to a method for extending the shelf-life of a food product comprising the step of incorporating a species of *Lactobacillus plantarum* into the food product, in which the species of *Lactobacillus plantarum* is selected from *Lb. plantarum (16)* NCIMB41875, or derivatives thereof, and *Lb. plantarum (62)* NCIMB41876, or derivatives thereof. Typically the food product is a fruit-based food product such as a fruit drink, or a dairy product. Preferably, the species of *Lb. plantarum* exhibits broad-spectrum anti-fungal activity.

The invention also relates to a fruit-based food product or a dairy product comprising at least one bacterial culture selected from the group consisting of:
- *Lb. plantarum (16)* NCIMB41875 or derivatives thereof; and
- *Lb. plantarum (62)* NCIMB41876, or derivatives thereof,

In a fifth aspect, the invention provides at least one bacterial culture selected from the group consisting of:
- *Lb. plantarum (16)* NCIMB41875 or derivatives thereof; and
- *Lb. plantarum (62)* NCIMB41876, or derivatives thereof,
for use as a medicament.

The invention also provides a starter culture for making a fermented dairy product comprising at least one bacterial culture selected from the group consisting of:
- *Lb. plantarum (16)* NCIMB41875 or derivatives thereof; and
- *Lb. plantarum (62)* NCIMB41876, or derivatives thereof,

### Brief Description of the Figures

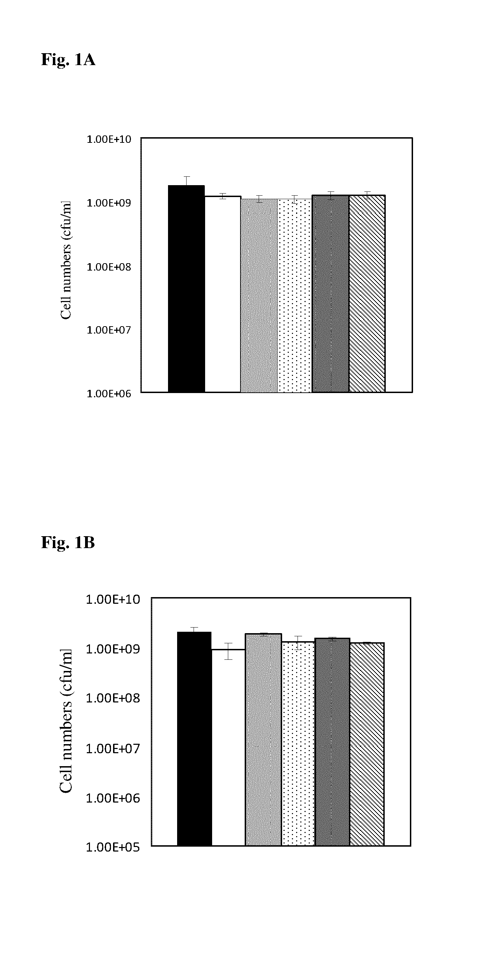

**Fig 1**. Effects of freeze drying on the survival of **(A)***Lb. plantarum* 16 and **(B)***Lb. plantarum* 62.
   Before freeze drying (■), one day post treatment (□), five days post treatment (▩), 14 days post treatment (▧), 22 days post treatment (▩), 28 days post treatment (▧).
**Fig 2****.** Osmotolerance of **(A)***Lb. plantarum* 16 and **(B)***Lb. plantarum 62.* 0 % NaCl (-◊-), 2.5 % NaCl (-●-), 5 % NaCl (-△-), 7.5 % (-▲-),10 % NaCl (-□-)

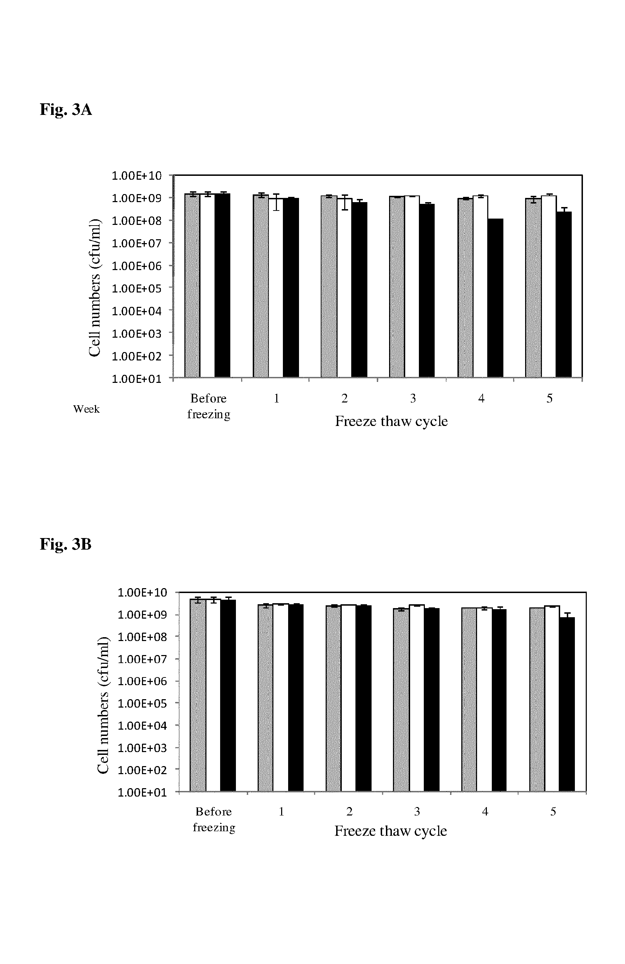

**Fig 3****.**Low temperature viability of **(A)***Lb. plantarum* 16 and **(B)***Lb. plantarum 62.* MRS broth (▩), 10 % RSM (□), 0.9 % NaCl (■).

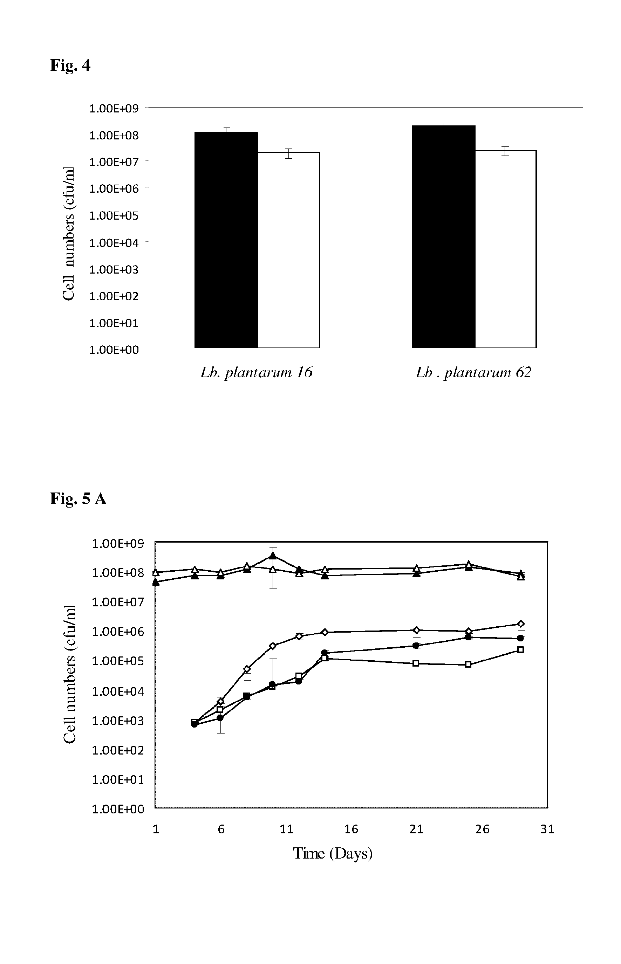

**Fig 4**. Effect of heat treatments on *Lb. plantarum* 16 and 62 in orange juice. Before heat treatment (■), after heat treatment (□).

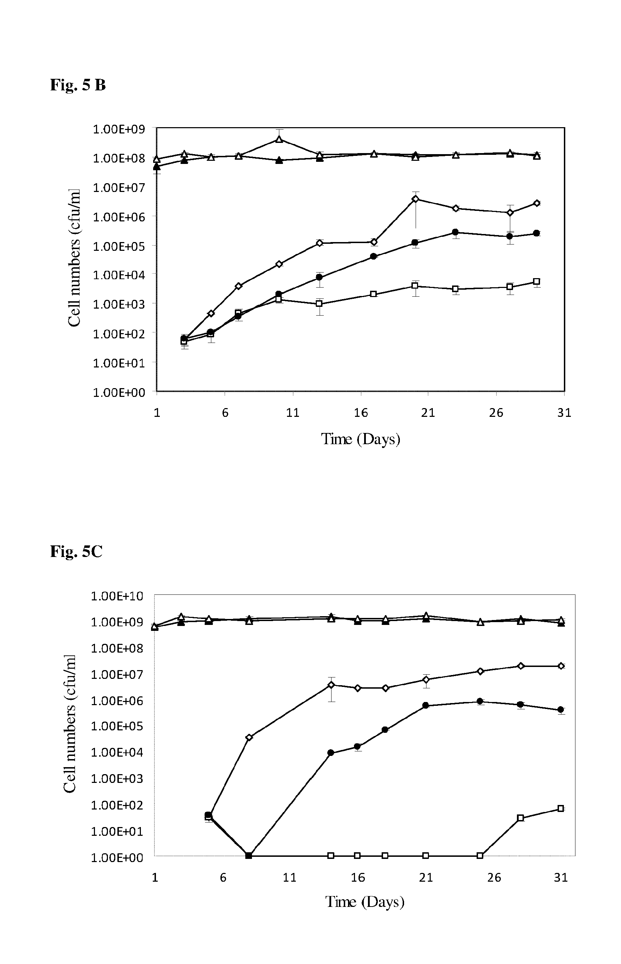

**Fig 5****.(A)** Effect of *Lb. plantarum* 16 and 62 on the growth of *R. mucilaginosa* in orange juice at 4 °C (high yeast inoculum). **(B)** Effect of *Lb. plantarum* 16 and 62 on the growth of *R*. *mucilaginosa* in orange juice at 4 °C (low yeast inoculum). (C) Effect of *Lb. plantarum* 16 and 62 on the growth of *R. mucilaginosa* in orange juice at 25 °C. *Lb. plantarum* 16 (cfu/ml) (-▲-), *Lb. plantarum* 62 (cfu/ml) (-△-), *R. mucilaginosa* control (-◊-), *R. mucilaginosa* and *Lb. plantarum* 62 (-●-), *R. mucilaginosa* and *Lb. plantarum* 16 (-□-).
**Fig 6****. (1)** Orange juice treated with *Lb. plantarum* 16. **(2)** Orange juice not treated with LAB.
**Fig 7**. Growth of *Lb. plantarum* 16 and 62 in 10% RSM. *Lb. plantarum* 16 (cfu/ml) (-□-), *Lb. plantarum* 62 (cfu/ml), (-▲-), *Lb. plantarum* 62 (pH) (-■-). *Lb. plantarum* 16 (pH) (-△-).

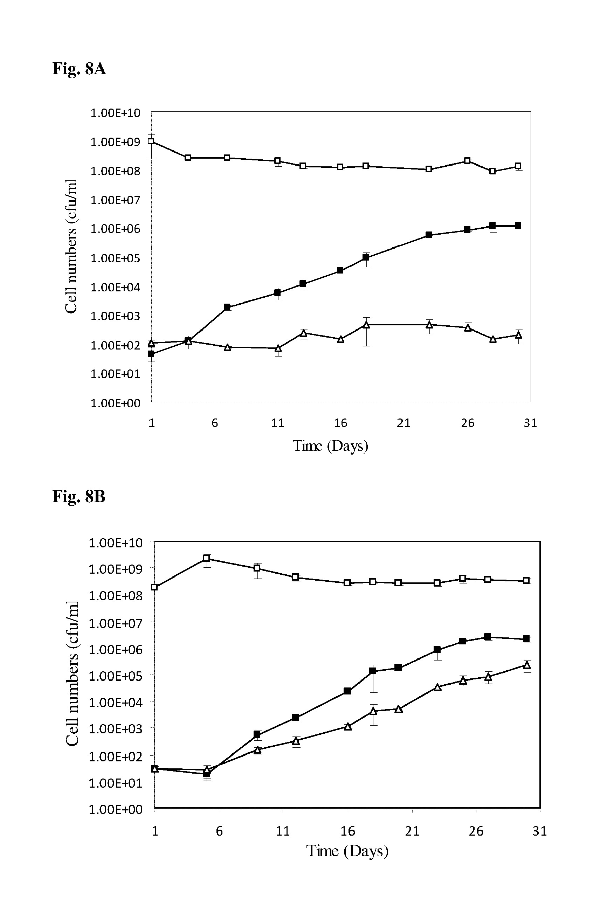

**Fig 8****: (A)** Effect of *Lb. plantarum* 16 on the growth of *R. mucilaginosa* in yoghurt at 4°C. *Lb. plantarum* 16 (-□-), *R. mucilaginosa* (-■-), *R. mucilaginosa* and *Lb. plantarum* 16 (-△-). **(B)** Effect of *Lb. plantarum* 62 on the growth of *R. mucilaginosa* in yoghurt at 4 °C. *Lb. planatrum* 62 (-□-),R. *mucilaginosa* (-■-), *R. mucilaginosa* and *Lb. plantarum* 62 (-△-).

### Detailed Description of the Invention

The term "derivatives" as applied to the deposited bacterial strain refer to bacteria which are derived from the deposited strain as a starting material and which have the same anti-fungal activity against the reference strain *Rhizopus stilonifer* DSMZ855 and *Fusarium culmorum* DSMZ1094 as the deposited strain when determined using the antifungal activity test provided below. Examples of derivatives include bacteria that are derived by genetically engineering the deposited strain to express a foreign gene, mutants of the deposited strain, or bacteria derived from deposited strain as a starting material by means of serial passage.

The term "food product prone to spoilage" means a food product (including a beverage) that is prone to spoilage due to the growth of a fungus. Example of such food products include fruit-based foods such as fruit drinks and fruit yoghurts, dairy products such as cheeses and yoghurts, and products that stored in a chilled environment.

The term "fruit-based food products" means fruit-containing foods and beverages such as fruit drinks (for example, orange, apple, pear, pineapple, mango, lemon, lime and banana drinks) including smoothies, and fruit-containing food products such as fruit-containing jellies, biscuits and pastries, cakes, scones, tins of fruit, and the like.

The term "dairy product" includes food products including beverages that are made from milk, for example butter, cheese, milk, milk-based drinks (i.e. flavoured milks, malts, shakes, etc). In particular, fermented dairy products are envisaged.

The term "for use as a medicament" as employed herein means that the composition is employed with a purpose of improving health, for example a food product that provides one or more health benefits or a pharmaceutical composition for a specific medical or veterinary indication, or a health supplement.

The composition, uses and methods of the invention are intended for use with mammals, but especially for use with humans and companion animals (for example dogs, cats and horses) and agricultural animals (for example bovine and porcine animals).

It will be appreciated that the isolated bacteria and bacterial cultures of the invention, or the derivatives thereof, may be in the form of live bacteria, dead bacteria or cellular components, fermentation broths, cell culture supernatants (i.e. cell free supernatants), or extracts of bacteria, fermentation broths, or supernatants. Suitably, the isolated bacteria or bacterial culture is provided in the form of a supernatant, ideally a cell free supernatant (CFS).

### Materials and methods

### Cultures and growth conditions

*Lb. plantarum* 16 and 62 are isolates from steep water and sauerkraut, respectively, and were cultivated in MRS broth for 24-48 hr at 30 °C under anaerobic conditions. A *R. mucilaginosa* strain was obtained from the UCC culture collection and was cultivated in sabouraud dextrose broth at 30 °C shaking for 16 hr.

### Screening for antifungal activity

LAB producing antifungal activity were identified using the overlay method (Mayr-Harting *et al.,* 1972). Selected LAB cultures were grown in MRS broth anaerobically for 48 hr at 30°C. Following this the LAB cultures were spotted in 5 µl quantities onto MRS agar (containing no cycloheximide) and grown anaerobically for 48 hr at 30°C. Fungal spore suspensions were prepared by scraping spores from the surface of the growing mould using a swab and resuspending the spores in quarter strength Ringers containing 0.8% Tween 80 (Kotan *et al.,* 2008). The MRS plates were overlaid with sabaroud dextrose semi-solid agar seeded with ~10⁵ - 10⁶ sporesml⁻¹ to which chloramphenicol (Sigma Aldrich) was added to a final concentration of 10 µg/ml to retard further bacterial growth. Plates were incubated at 30°C for 48 hr after which zones of inhibition surrounding the LAB colony were measured. Zones were measured and scored against the following arbitrary scale: no inhibition observed: -, 1-5 mm zone of inhibition: +, 6-10 mm zone of inhibiton: ++, 11-15 mm zone of inhibition: +++, ≥ 16mmzone of inhibition: ++++. Screening was performed against *Pen. expansum* due to its observed antifungal sensitivity and suitable growth characteristics (Hassan and Bullerman, 2008).

### 1.4Antifungal activity spectrum

*Lb. plantarum* 16 and *Lb. plantarum* 62were then screened for anti-fungal activity, using the overlay technique as described above and using the following indicators: *Rhizopus stilonifer* (DSMZ855) and *Fusarium culmorum* (DSMZ1094).

### Effect of freeze drying on viability of Lb. plantarum16 and 62

The effect of freeze drying on the viability of *Lb. plantarum* 16 and 62 was assessed over a 4 week period. Each isolate was grown for 48 hr in MRS broth and viable cell counts performed to determine cell numbers before freeze drying. Serial dilutions were performed in quarter-strength Ringers with appropriate dilutions plated onto MRS agar. 1 ml samples of the 48 hr culture were taken and cells were collected by centrifugation at 5,000 rpm for 15 min, washed twice in quarter-strength Ringers and finally resuspended in 10 % reconstituted skimmed milk (RSM) to a final volume of 10 ml. The cultures were freeze-dried overnight in a Labconco Freezone 6 freeze drier and rehydrated to the original volume in 10 % RSM. The lyophilised cells were consequently stored at - 80 °C. Viable cell counts were performed after freeze treatment at regular intervals over a 4 week period on MRS agar at 30 °C for 24 - 48 hr.

### Osmotolerance

The osmotolerance of *Lb. plantarum* 16 and 62 was assessed using sodium chloride (NaCl) concentrations of 0, 2.5, 5.0, 7.5 and 10 %. Each isolate was inoculated into MRS broth supplemented with the appropriate NaCl concentration. Viable cell counts were performed at regular intervals over a 24 hr period on MRS agar.

### Low temperature viability

The ability of *Lb. plantarum* 16 and 62 to survive low temperatures was examined adapted from *Sheehan et al.,* 2006. Viability was examined in 10 % Reconstituted Skimmed Milk (RSM), MRS broth and a 0.9 % sodium chloride solution. Briefly, each isolate was grown overnight in MRS broth and 1.5 ml of culture was harvested by centrifugation at 4,000 rpm for 10 min. Cells were washed twice in quarter strength Ringers and finally resuspended to the original volume in either MRS broth, 10 % RSM or 10 mM sodium chloride solution. Samples were stored at -20 °C until further use. Viable cell counts were performed on MRS agar before freezing and subsequently after every freeze thaw cycle.

### Comparison of antifungal LAB to commercial chemical preservatives

The cell-free supernatant of both isolates were compared to commercially available preservatives according to Yang and Chang *et al.* (2010). *Lb. plantarum* 16 was grown anaerobically in 200 ml MRS broth for 48 hr at 30 °C anaerobically. Cells were harvested by centrifugation at 10,000 rpm for 20 min. The supernatant was freeze dried overnight in a Labconco Freezone 6 freeze drier. The resulting freeze dried product was concentrated 20 times relative to the original volume using 20 mM sodium acetate (pH 4.0). The following commercial preservatives were used: sodium benzoate (0.1, 0.5 and 1.0 %), potassium sorbate (0.1, 0.5 and 1.0 %), acetic acid (1.0, 3.0 and 5.0 %), benzoic acid (0.1 and 0.5 %), calcium lactate (1.0, 2.0 and 3.0 %) and calcium propionate (1.0, 2.0 and 3.0 %) at the following concentrations. The concentrated supernatant was compared to the commercial preservatives using the paper disk assay. Briefly, sterile paper discs (Sigma) were soaked in concentrated CFS or test preservative and placed onto sabouraud dextrose agar that had been seeded with *Penicillium expansum* (10⁴ cfu/ml). The plates were incubated at 30 °C for 48 hr aerobically and subsequently examined for zones of clearance surrounding the sterile disks.

### API 50CH carbohydrate fermentation patterns

Carbohydrate utilisation of both isolates was analysed using the API 50CH kit and performed according to manufacturer's instructions. After inoculation of the bacterial suspension into the test panels the kits were incubated at 30 °C and results were read after 24 and 48 hr incubation periods.

### Preparation of strains for inoculation into orange juice

*Lb. plantarum* 16 and 62 were grown in MRS broth for 48 hr under anaerobic conditions. 1 ml of the 48 hr cultures was harvested at 4,000 rpm for 15 min. The cells were washed twice in quarter-strength Ringers and then resuspended in the same volume of orange juice.

### Effects of heat treatment on the survival of antifungal LAB in orange juice

The effect of thermal treatments commonly employed in the food industry on the viability of *Lb. plantarum* 16 and 62 was investigated in orange juice according to Sheehan *et al.,* 2007. 1 ml volumes of orange juice containing approx. 10⁷ cfu/ml of *Lb. plantarum* 16 or 62 were exposed to heat treatments of either 90 °C for 1 min or 76 °C for 30 sec in a water bath. Viable cell counts were performed before and after each heat treatment on MRS agar.

### Inoculation and storage of orange juice

A commercially available orange juice was obtained for the purpose of the trial. 1 ml volumes of washed cells were inoculated into 40 ml volumes of orange juice to give a final concentration of approx. 10⁷/10⁸cells/ml of orange juice. Each orange juice was set up in triplicate and stored at 4 °C for a period of 4 weeks. After 9 days, each of the orange juice samples was challenged with the yeast spoiler *R. mucilaginosa* to a final concentration of 10² cfu/ml. Viable cell counts were performed at regular intervals over the 4 week period to determine both LAB and yeast levels in the orange juice. LAB were recovered on MRS agar at 30 °C for 24 - 48 hr and *R. mucilaginosa* was enumerated on sabouraud dextrose agar at 30 °C for 24 - 48 hr. 40 ml of orange juice inoculated with *R. mucilaginosa* (at a level of 10² cfu/ml) was used as a control.

### Growth in 10% Reconstituted Skimmed Milk (RSM)

*Lb. plantarum* 16 and 62 were grown in 10 % RSM for 48 hr at 30 °C. A 1 % inoculum was used to inoculated fresh 10 % to give a final concentration of approx. 10⁵ cfu/ml. Both cell numbers and pH levels were monitored at intervals over a 6 day period at 30 °C. Each sample was assessed in triplicate.

### Yoghurt production

A small-scale production of yoghurt was used to assess the role of *Lb. plantarum* 16 and 62 as antifungal adjuncts. 40 ml volumes of 10 % RSM was treated at 105 °C for 10 min. *Streptococcus thermophilus* ST00111 and *Lactobacillus delbrueckii* subsp. *bulgaricus*CH2 were inoculated at 1 %, while *Lactobacillus plantarum* was inoculated to give a final concentration of ~10⁸ cfu/ml. Fermentation was continued at 37 °C for 4-5 hr until a pH of 4.6 was reached, after which the resulting yoghurts were stored at 4 °C. After 16 hours storage at 4 °C each of the yoghurts were challenged with *R. mucilaginosa* to give a final concentration between 10¹ and 10² cfu/ml. Control yoghurts were also prepared without *Lb. plantarum* 16 and 62. LAB were enumerated on MRS agar and yeast cells recovered on sabouraud dextrose agar supplemented with 10 µg/ml chloramphenicol.

### Results

### Anti-fungal activity of Lb. plantarum isolates

Both *Lb. plantarum* 16 and *Lb. plantarum* 62 were found to have +++ activity (11-15mm zone of inhibition) against the mould *R.stolonifer* DSMZ855 and ++++ activity (≥ 16mm zone of inhibition) against *F. culmorum* DSMZ1094.

### Effect of freeze-drying on viability of Lb. plantarum16 and 62

The viability of both *Lb. plantarum* isolates after exposure to freeze-drying was investigated. After a period of 4 weeks storage as lyophilised cells at - 80 °C the viability of *Lb. plantarum* 16 decreased from 1.8 x 10⁹ cfu/ml to 1.26 x 10⁹ cfu/ml showing a 70 % survival rate. *Lb. plantarum* 62 exhibited a 60 % survival after 4 weeks storage.

### Osmotolerance

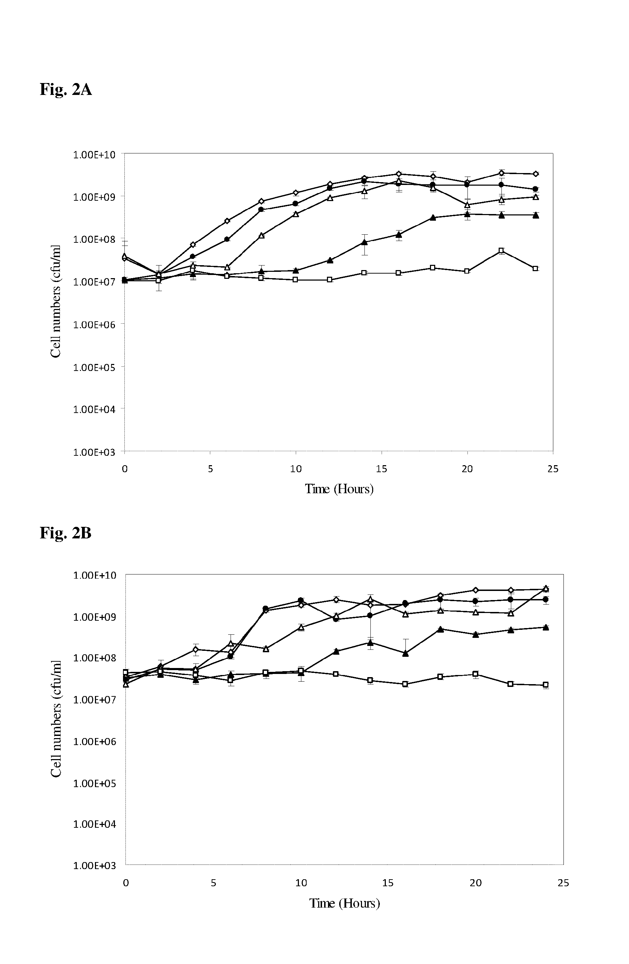

Both strains showed high viability in MRS supplemented with a sodium chloride concentration as high as 10 %. *Lb. plantarum* 16 was found to be slightly more osmotolerant reaching levels of 1.9 x 10⁷ cfu/ml in 10 % sodium chloride compared to *Lb. plantarum* 62. 10 % sodium chloride concentrations were found to exert a bacteriostatic effect upon both strains (Fig. 2). The ability to survive such an osmotic challenge makes these strains versatile for application in dried or salted foods.

### Low temperature viability

The viability of each isolate after five freeze-thaw cycles was evaluated in three types of media. Both strains showed high levels survival in 10% RSM and this appeared to be the optimal cryoprotectant for both strains. *Lb. plantarum* 16 cell numbers decreased from 1.5 x 10⁹ cfu/ml to 1.3 x 10⁹ cfu/ml in 10 % RSM showing a survival rate of 87 % compared to a 48 % survival rate for *Lb. plantarum* 62. 0.9 % sodium chloride solution proved to have the least protective properties with only 15.4 % of *Lb. plantarum* 16 cells surviving, however *Lb. plantarum* 62 showed a higher survival rate of 33.4 %. Finally cell numbers remained relatively stable following freeze thaw treatment in MRS broth. Viable cell numbers decreased from 1.5 x 10⁹ cfu/ml to 9.0 x 10⁸ cfu/ml for *Lb. plantarum* 16, while *Lb. plantarum* 62 viable counts were found to be 2.0 x 10⁹ cfu/ml from an initial number of 4.6 x 10⁹ cfu/ml.

### Commercial preservatives

The antifungal activity contained within concentrated cell-free supernatants (CFS) from both strains was compared with that exhibited by commercially available chemical preservatives. *Pen. expansum* and *R. mucilaginosa* were selected as target fungi. Concentrated supernatants of both strains were found to as effective as 5 % acetic acid, 0.5 % potassium sorbate and 1 % sodium benzoate. Additional preservatives such as calcium propionate, benzoic acid, and calcium lactate were also examined. Under the conditions tested, the latter preservatives (at concentrations up to 3%) were found to be less effective as an antifungal agent than the concentrated CFS from the two *Lb. plantarum* strains (data not shown).

### Carbohydrate utilisation

Both isolates were found to have identical carbohydrate fermentation profiles with each strain having 99.2 % identity to the type strain of *Lb. plantarum.* Of the 49 carbohydrates analysed the strains were capable of utilising 23. Both isolates utilised the same carbohydrates. See Table 1 below.

### Effects of heat treatment on the survival of LAB in orange juice

Pasteurisation conditions of 90 °C for 1 min were found to completely inactivate the isolates and therefore milder heat treatments were investigated to assess the survival capabilities of the two antifungal strains. Following treatment at 76 °C for 30 sec *Lb. plantarum* 16 cell numbers decreased from 1.14 x 10⁸ cfu/ml to 2.05 x 10⁷ cfu/ml showing a viability loss of 82.4 %. *Lb. plantarum* 62 presented similar findings with an approximately one log reduction in viable cell number.

### Orange juice trials

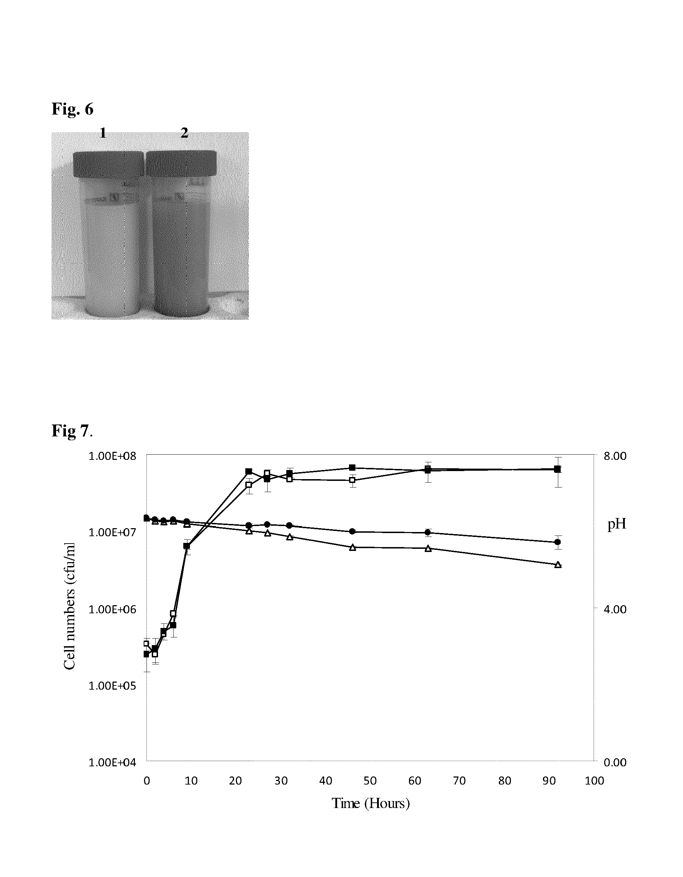

The ability of both antifungal isolates to increase the shelf-life of orange juice was investigated in two different trials. Varying inoculation levels and storage temperatures were examined during the course of the trials. Firstly the effects of two different storage temperatures were examined. *Lb. plantarum* 16 exerted the best protective properties against *R. mucilaginosa* when inoculated into orange juice stored at 25 °C. At room temperature yeast levels in the control juice reached 1.9 x 10⁷ cfu/ml. The orange juice seeded with *Lb. plantarum* 16 caused a five log reduction in yeast cell numbers when the antifungal isolate was introduced at a final concentration of 10⁹ cfu/ml. A two log reduction in *R. mucilaginosa* levels was observed in the orange juice containing the antifungal *Lb. plantarum* 62 seeded at a level of 10⁸ cfu/ml. After 30 days storage at room temperature there was a noticeable difference in the appearance of the control juice as compared to the juice containing the protective cultures (Fig. 6). The control sample showed visible levels of spoilage causing the juice to discolour compared to the sample juices containing *Lb. plantarum* 16 and 62 which retained the original colour throughout the trial.

The effect of varying inoculation levels of 10¹ and 10² cfu/ml on the protective capabilities of both isolates was also examined in orange juice stored at 4 °C. *R. mucilaginosa* levels reached 1.7 x 10⁷ cfu/ml in the control juice not containing any protective cultures. When the juice was challenged with yeast levels of 5 x 10² cfu/ml at 4 °C *Lb. plantarum* 16 reduced the contaminant levels by one log. However, only a partial decrease in yeast cell numbers was observed in the juice inoculated with *Lb. plantarum* 62 compared to the control. The effect of a lower level of *R. mucilaginosa*(5 x 10¹ cfu/ml) was also investigated in a separate trial. In the juice containing *Lb. plantarum* 16 (1 x 10⁸ cfu/ml) the numbers of viable yeast cells reached a level that was three log less than that found in juice without the antifungal LAB.

The juice containing *Lb. plantarum* 62 elicited a 10-fold reduction in the viable count of *R*. *mucilaginosa* as compared to the control. Optimal inhibition was observed when the juice was stored at room temperature with LAB levels of 1 x 10⁹ cfu/ml and a contamination level of 5-6 x 10¹ cfu/ml.

### Growth in 10% Reconstituted Skimmed Milk (RSM)

The ability of both isolates to grow in 10 % RSM was investigated. Both strains were inoculated to a final viable count between 2-3 x 10⁵ cfu/ml. A two log increase in viable cell numbers was observed for both strains over a seven day period at 30 °C. *Lb. plantarum* 16 reached cell numbers of 4.9 x 10⁷ cfu/ml compared to *Lb. plantarum* 62 which reached 8.7 x 10⁷ cfu/ml. Final pH levels of 4.6 and 5.5 were observed for *Lb. plantarum* 16 and 62, respectively, which represents a significant drop from the initial pH value of the medium(pH = 6.33).

### Yoghurt production

The anti-yeast activity of both strains was also investigated in a yoghurt-based model employing *R. mucilaginosa* as the spoilage organism. Both *Lb. plantarum* strains maintained good viability in the yoghurt during four weeks storage at 4 °C with levels of approx. 10⁸ cfu/ml maintained throughout the trial. The control yoghurt (fermented without any antifungal LAB) contained 1.1 x 10⁶ cfu/ml of yeast cells after 30 days storage at 4°C. The yoghurt supplemented with *Lb. plantarum* 16 demonstrated little increase in yeast levels compared to the numbers detected in the control yoghurt. On day one of the trial a yeast viable count of 1.3 x 10² cfu/ml was determined, while following 30 days of storage yeast levels of 2.1 x 10² cfu/ml were seen, showing little increase in viable cell numbers throughout the four week storage period. *Lb. plantarum* 62 also showed a partial decrease in yeast cell numbers, with a 10-fold reduction compared to the control yoghurt.

The invention is not limited to the embodiments hereinbefore described which may be varied in construction and detail without departing from the spirit of the invention.

**Table 1. Api 50CH fermentation patterns for Lb. plantarum 16 and 62.**

| **Carbohydrate** | | ***Lactobacillus plantarum* 16** | ***Lactobacillus plantarum 62*** |
|---|---|---|---|
| | 1. Glycerol | - | - |
| | 2. Erythritol | - | - |
| | 3. D arabinose | - | - |
| | 4. L arabinose | - | - |
| | 5. Ribose | + | + |
| | 6. D xylose | - | - |
| | 7. L xylose | - | - |
| | 8. Adonitol | - | - |
| | 9. β methyl-D xyloside | - | - |
| | 10. Galactose | + | + |
| | 11. Glucose | + | + |
| | 12. Fructose | + | + |
| | 13. Mannose | + | + |
| | 14. Sorbose | - | - |
| | 15. Rhamnose | - | - |
| | 16. Dulcitol | - | - |
| | 17. Inositol | - | - |
| | 18. Mannitol | + | + |
| | 19. Sorbitol | + | + |
| | 20. α-Methyl-D mannoside | - | - |
| | 21. α-Methyl-D glucoside | - | - |
| | 22. N acetyl glucosamine | + | + |
| | 23. Amygdalin | + | + |
| | 24. Arbutin | + | + |
| | 25. Esculin | + | + |
| | 26. Salicin | + | + |
| | 27. Cellobiose | + | + |
| | 28. Maltose | + | + |
| | 29. Lactose | + | + |
| | 30. Melibiose | + | + |
| | 31. Sucrose | + | + |
| | 32. Trehalose | + | + |
| | 33. Inulin | + | + |
| | 34. Melezitose | + | + |
| | 35. Raffinose | - | - |
| | 36. Starch | - | - |
| | 37. Glycogen | - | - |
| | 38. Xylitol | - | - |
| | 39. Gentiobiose | + | + |
| | 40. D turanose | - | - |
| | 41. D lyxose | - | - |
| | 42. D tagatose | - | - |
| | 43. D fucose | - | - |
| | 44. L fucose | - | - |
| | 45. D arabitol | + | + |
| | 46. L arabitol | - | - |
| | 47. Gluconate | + | + |
| | 48. 2 keto gluconate | - | - |
| | 49. 5 keto gluconate | - | - |

## Claims

1. An isolated bacterial culture selected from the group consisting of the following bacteria deposited with the National Collection of Industrial and Marine Bacteria Limited (NCIMB) on 19 October 2011:
- *Lb. plantarum (16)* NCIMB41875, or derivatives thereof; and
- *Lb. plantarum (62)* NCIMB41876, or derivatives thereof,
or a fermentation broth or supernatant thereof, or an extract of the broth, supernatant or bacterial culture.

2. A bacterial culture of Claim 1, or a fermentation broth or supernatant thereof, or an extract of the broth, supernatant or bacterial culture, for use as an anti-fungal agent.

3. A bacterial culture of Claim 1, or a fermentation broth or supernatant thereof, or an extract of the broth, supernatant or bacterial culture, for use as a preservative in a food product prone to spoilage.

4. A bacterial culture of Claim 1, or a fermentation broth or supernatant thereof, or an extract of the broth, supernatant or bacterial culture, for use as a preservative in a fruit-based food product.

5. A bacterial culture of Claim 1, or a fermentation broth or supernatant thereof, or an extract of the broth, supernatant or bacterial culture, for use as a preservative in a fruit juice.

6. A bacterial culture of Claim 1, or a fermentation broth or supernatant thereof, or an extract of the broth, supernatant or bacterial culture, for use as a preservative in a dairy product.

7. A bacterial culture of Claim 1 for use in a starter culture for a fermented dairy product.

8. A bacterial culture of Claim 1, or a fermentation broth or supernatant thereof, or an extract of the broth, supernatant or bacterial culture, for use as a medicament.

9. A method for extending the shelf-life of a food product comprising the step of incorporating an isolated bacterial culture of Claim 1, or a fermentation broth or supernatant thereof, or an extract of the broth, supernatant or bacterial culture, into the food product.

10. A method as claimed in Claim 8 in which the food product is a fruit-based product or a dairy product.
